# EUROPEAN PATENT APPLICATION

(11) **EP 0 914 816 A1**
(43) Date of publication of application: **12.05.1999**
(21) Application number: 98919625.8
(22) Date of filing: 15.05.1998
(51) Int. Cl.: A61K 7/06, A61K 35/78

(54) **SCALP CARE COMPOSITIONS**

(30) Priority: 19.05.1997 JP 144704/97; 16.07.1997 JP 207246/97; 16.07.1997 JP 207248/97; 12.09.1997 JP 268070/97; 07.11.1997 JP 322093/97
(71) Applicant: SHISEIDO COMPANY LIMITED, Chuo-ku Tokyo 104-10 (JP)
(72) Inventor: SHIBATA, Yuki, Shiseido Research Center 1, Yokohama-shi, Kanagawa 223-8553 (JP); IDETA, Ritsuro, Shiseido Res. Cter 2, Shiseido, Yokohama-shi, Kanagawa 236-0004 (JP); SOMA, Tsutomu, Shiseido Res. Cter 2, Shiseido, Yokohama-shi, Kanagawa 236-0004 (JP); MORIYA, Yoshiki, Shiseido Res. Cter 1, Shiseido, Yokohama-shi, Kanagawa 223-8553 (JP); TSUJI, Yoshiharu, Shiseido Res. Cter 1, Shiseido, Yokohama-shi, Kanagawa 223-8553 (JP); KOJIMA, Nao, Chuo-ku, Tokyo 104-8010 (JP); IFUKU, Ohji, Shiseido Res. Cter 2, Shiseido, Yokohama-shi, Kanagawa 236-0004 (JP)
(74) Representative: TER MEER STEINMEISTER & PARTNER GbR
(86) International application number: JP9802162
(87) International publication number: WO9852516

(57) **Abstract**

A composition for use on the scalp, a composition for promoting tyrosinase activity, and a composition for preventing gray hair which characteristically contain extracts from various plants.

The composition for use on the scalp of the present invention can effectively prevent dandruff and is safe. Also, the composition for use on the scalp of the present invention is particularly effective in preventing and treating dandruff which is generated due to scalp stimulation by surfactants such as shampoos, rinses, etc. It also is highly effective at preventing scalp itching.

Also, the present invention provides a composition with a superior tyrosinase activity promoting action and gray hair prevention action.

## Description

### FIELD OF THE INVENTION

The present invention relates in general to a composition for use on the scalp, and more particularly to a composition for use on the scalp which has a superior dandruff control effect.

Also, the present invention is useful as a composition for promoting tyrosinase activity and a composition for preventing gray hair.

### BACKGROUND OF THE INVENTION

[1] Generally, dandruff consists of the secretions of sebaceous glands, secretions of sweat glands and particles peeled-off or desorbed from the epidermis layer. Dandruff is usually generated due to accelerated secretion from sebaceous glands and such. It is said that dandruff generation is pathologically promoted when the skin is infected with bacteria and/or yeast. For this reason, compositions for use on the scalp which contain antimicrobial agents or disinfectants have been used conventionally.
   However, some disinfectants and/or antimicrobial agents have problems with regard to use on human bodies due to skin irritation. Also, it has been pointed out that the compositions for use on the scalp containing disinfectants and/or antimicrobial agents, when used continuously, disrupt the delicate balance of resident bacteria on the skin, occasionally inducing undesirable conditions from the so-called ecological point of view.
   For example, zinc pyrithione (ZPT), which has been regarded as most effective as a dandruff prevention drug, is categorized as an antimicrobial agent and it is said that the use of a large amount of this drug should be avoided as much as possible.
   The present invention was carried out based on the aforementioned problems in the conventional technology and its object is to provide a composition for use on the scalp which has effective dandruff control actions and also is safe for the scalp.
   In order to achieve she aforementioned object, the inventors conducted earnest research to obtain a compound which effectively prevents dandruff when used on the scalp and is superior in terms of safety, and discovered that extracts from certain plants are very effective against prolific thickening of the epidermis, dryness accompanied by erythema and desquamatory changes.
[2] As a composition for preventing gray hair, various compounds (or substances) have been proposed for use which were obtained by screening for effective substances based on the mechanism of gray hair occurrence or, the other way around, based on the mechanism of melanin pigment formation, or by randomly checking the gray hair preventive actions of various substances.
   Examples include one which attempts to improve the cyclic AMP production capacity of melanocytes (Japanese unexamined patent publication Tokkai Hei 4-124122), one which attempts to activate melanin production by melanocytes (Tokkai Hei 5-78222, Tokkai Hei 7-285874, Tokkai Hei 7-316026, etc.) and one which claims to have both a hair restoration effect and a gray hair preventive effect (Tokkai Hei 7-112918, Tokkai Hei 7-126129). Most of them have as an effective ingredient an extract from animals and/or plants. They are described as having the ability to achieve a certain objective.
   However, considering the complexity of the gray hair occurrence mechanism and, on the other hand, diversity of natural products, it is desirable to provide a new composition for preventing gray hair which uses an additional variety of ingredients derived from natural products.
   Therefore, the main object of the present invention is to provide a composition for preventing gray hair which, although conventionally not used to prevent gray hair, exhibits a superior effect in actual use than those plant ingredients which have been used (or have been proposed to be used) to prevent gray hair
   In order to solve the aforementioned problem, the inventors conducted basic research on the gray hair occurrence mechanism and the effects of various compounds or substances on each stage of the mechanism, and also investigated their efficacy in actual use.
   As a result, the inventors discovered that an extract from a plant of the Apocynaceae family, genus Parameria, has superior tyrosinase promoting actions and gray hair preventive (or gray hair blackening) actions.
   The inventors also discovered that plant extracts derived from the Ericaceae family, genus Arctostaphylos, the Chenopodiaceae family, genus Chenopodium and genus Poterium and the Ericaceae family, genus Gautheria, which are easily procurable in large quantities, have superior tyrosinase promoting actions and gray hair preventive (or gray hair blackening) actions.
   The inventors also discovered that a plant extract from guaco misto (scientific name: Mykania Glomerata) of the Compositae family, genus Mykania, which are easily procurable in large quantities, have superior tyrosinase promoting actions and gray hair preventive (or gray hair blackening) actions.
   The inventors also discovered that a Japanese pepper extract has the desired tyrosinase activity promoting effect, thus completing the present invention.
   The composition for promoting tyrosinase activity can be used not only for preventing gray hair but also for self tanning and such.

### DISCLOSURE OF THE INVENTION

[1] That is, the present invention is a composition for use on the scalp which characteristically contains one or more types of extracts chosen from among extracts of the following plants.
   (1) Kayu rapet (scientific name: Parameria laevigata)
   (2) Kemukus (scientific name: Piper cubeba)
   (3) Tempuyung (scientific name: Sonchus arvensis L.)
   (4) Belantas (scientific name: Pluchea indica L.)
   (5) Mesoyi (scientific name: Massoia aromatica Becc.)
   (6) Pule (scientific name: Alstonia scholaris)
   (7) Pulowaras (scientific name: Alycia reindwartii BI.)
   (8) Sintok (scientific name: Cinnamomum sintoc BI.)
[2] That is, the present invention relates to a composition for use on the scalp, a composition for promoting tyrosinase activity, and/or a composition for preventing gray hair which contains as an effective ingredient an extract from a plant of the Apocynaceae family, genus Parameria.
   In the aforementioned composition, Parameria laevigata (Kayu rapet) is preferably used as the plant of genus Parameria.
[3] That is, the present invention relates to a composition for use on the scalp, a composition for promoting tyrosinase activity, and/or a composition for preventing gray hair which contains as an effective ingredient a plant extract wherein the effective ingredient is at least one type of extract derived from a plant of a genus chosen from a group consisting of the Ericaceae family, genus Arctostaphylos, the Chenopodiaceae family, genus Chenopodium and genus Poterium and the Ericaceae family, genus Gautheria.
   In the aforementioend invention, it is preferable to use Pinguica (scientific name: Arctostaphylos Pugens) for the plant
   of the Ericaceae family, genus Arctostaphylos, Aritaso (in Japanese; scientific name: Chenopodium Ambrosioides L.) for the plant of the Chenopodiaceae family, genus Chenopodium, Zapote (scientific name: Poterium Zapote) for the plant of genus Poterium, and Axcopaque (scientific name: Gautheria Acuminata) for the plant of the Ericaceae family, genus Gautheria.
[4] That is, the present invention relates to a composition for use on the scalp, a composition for promoting tyrosinase activity, and/or a composition for preventing gray hair which contains as an effective ingredient a plant extract wherein the effective ingredient is guaco misto (scientific name: Mykania Glomerata) of the Compositae family, genus Mykania.
[5] That is, the present invention relates to a composition for use on the scalp, a composition for promoting tyrosinase activity, and/or a composition for preventing gray hair which contains as an active ingredient a Japanese pepper extract.
   In the present invention, the phrase "preventing gray hair" can also mean "blackening gray hair".

### THE BEST MODES OF THE EMBODIMENTS

The configuration of the present invention is described in detail below.
[1] The inventions of claim 1 and claim 2
   The plants used in the present invention grow particularly in dry grasslands and pastures in Indonesia. The extract used in the present invention is obtained by immersing or heated refluxing the leaves, stems, fruits, flowers, bark, seeds, fruits or the whole plant of the aforementioned plant in an extraction solvent, followed by filtering and condensation. The extraction solvent used in the present invention can be any solvent which is normally used for extraction. Examples include alcohols such as methanol and ethanol, hydrated alcohols, and organic solvents such as acetone and ethyl acetate, and they can be used either independently or in combination.
[2] The inventions of claims 3 through claim 8
   For the plant of the Apocynaceae family, genus Parameria, used in the present invention, any plant species can be used as long as it belongs to this genus and is suitable for the object of the present invention. A representative example is Parameria laevigata which is commonly called Kayu rapet.
   In the present invention, the plant of genus Parameria can be used either raw or dried. However, in terms of usability and pharmaceutical preparation, it is preferable to use it in the form of dried powder or a solvent extract. Regarding the part to be used, leaves, stems, flowers, barks seeds or fruits as well as the whole plant of the plant of genus Parameria can be used, but it is preferable to use the whole plant, leaves or seeds.
   The extract of the plant of genus Parameria can be obtained with a conventional method. For example, it can be obtained by drying the plant of genus Parameria, if necessary, immersing it in an extraction solvent for a certain amount of time or bringing it into contact with a heat-refluxing extraction solvent, followed by filtration and concentration. For the extraction solvent, any solvent normally used for extraction can be used. For example, alcohols including methanol, ethanol, propylene glycol, 1,3-butylene glycol and glycerine, organic solvents including chloroform, dichloroethane, carbon tetrachloride, acetone and ethyl acetate can be used individually or in combination. The extract obtained by the extraction using the aforementioned solvents can be used as it is, or it can be concentrated before use, or the concentrated extract can be treated with the adsorption method which uses an ion exchange resin and such to remove impurities, or the extract can be adsorbed to a column of a porous polymer (such as Amberlite XAD-2) and then eluted with methanol or ethanol, followed by concentration before use. An extract obtained by the distribution method using water/ethyl acetate and such for extraction can also be used.
[3] The inventions of claims 9 through claim 14
   For the plant of the Ericaceae family, genus Arctostaphylos, to be used in the present invention, any plant species can be used as long as it belongs to this genus and is suitable for the object of the present invention. A representative example is Pinguica (scientific name: Arctostaphylos Pugens). Regarding the part to be used of these plants for extraction, the leaves, stems, flowers, bark, seeds or fruits as well as the whole plant can be used, but it is preferable to use the whole plant, leaves or seeds.
   For the plant of the Chenopodiaceae family, genus Chenopodium, to be used in the present invention, any plant species can be used as long as it belongs to this genus and is suitable for the object of the present invention. A representative example is Aritaso (in Japanese; scientific name: Chenopodium Ambrosioides L.). Regarding the part to be used of these plants for extraction, the leaves, stems, flowers, bark, seeds or fruits as well as the whole plant can be used, but it is preferable to use the whole plant, leaves or seeds.
   For the plant of genus Poterium, to be used in the present invention, any plant species can be used as long as it belongs to this genus and is suitable for the object of the present invention. A representative example is Zapote (scientific name: Poterium Zapote). Regarding the part to be used of these plants for extraction, leaves, stems, flowers, bark, seeds or fruits as well as the whole plant can be used, but it is preferable to use the whole plant, leaves or seeds.
   For the plant of the Ericaceae family, genus Gautheria, to be used in the present invention, any plant species can be used as long as it belongs to this genus and is suitable for the object of the present invention. A representative example is Axcopaque (scientific name: Gautheria Acuminata). Regarding the part to be used of these plants for extraction, leaves, stems, flowers, bark, seeds or fruits as well as the whole plant can be used, but it is preferable to use the whole plant, leaves or seeds.
   According to the present invention, each extract can be used independently or in combination. In general, the extract used in the present invention can be obtained by drying the plant, if necessary, immersing it in an extraction solvent for a certain amount of time or bringing it into contact with a heat-refluxing extraction solvent, followed by filtration and concentration. Selection of the extraction solvent is not limited. Examples include alcohols such as methanol and ethanol, 1,3-butylene glycol, acetone and ethyl acetate as well as a mixture thereof.
[4] The inventions of claims 15 through claim 17
   Regarding the part to be used of guaco misto (scientific name: Mykania Glomerata) of the Compositae family, genus Mykania, for extraction in the present invention, the leaves, stems, flowers, bark, seeds or fruits as well as the whole plant can be used, but it is preferable to use the whole plant, leaves or seeds.
   In general, the extract used in the present invention can be obtained by drying the plant, if necessary, immersing it in an extraction solvent for a certain amount of time or bringing it into contact with a heat-refluxing extraction solvent, followed by `filtration and concentration. Selection of the extraction solvent is not limited. Examples include alcohols such as methanol and ethanol, 1,3-butylene glycol, acetone and ethyl acetate as well as a mixture thereof.
[5] The inventions of claims 18 through claim 20
   The Japanese pepper extract used as the effective ingredient in the tyrosinase activity promoting agent of the present invention is an extract from the Japanese pepper (Zanthoxylum piperitum (L.) DC) of the Rutaceae family, genus Zanthoxylum. The fruits (fruit skin), flowers, leaves and bark of this plant can be used as the raw material of this extract. It is particularly preferable to use a Japanese pepper extract whose raw material is the fruits (fruit skin) as the effective ingredient of the tyrosinase activity promoting agent of the present invention.
   This Japanese pepper extract can be obtained by a method generally used to obtain an extract from a plant.
   That is, the extract can be obtained by conducting solvent extraction of the intact or crushed raw material, after drying it if necessary. For the extraction solvent used for this process, any solvent normally used for extraction of ingredients from a plant can be used and the selection is not particularly limited.
   Examples include: hot water or water; lower alcohols including methanol, ethanol, isopropanol and n-butanol; polyhydric alcohols including propylene glycol and 1,3-butylene glycol; hydrates of these alcohols; and hydrocarbon type solvents such as n-hexane and toluene. It is preferable to use a lower alcohol such as methanol or ethanol for the extraction solvent. When using these lower alcohols for the extraction solvent, it is possible to blend the obtained extract as an effective ingredient in the tyrosinase activity promoting agent of the present invention, but it is also possible to distill away the extraction solvent and, after drying if necessary, blend it in.
   In place of blending the Japanese pepper extract obtained by the aforementioned process as an effective ingredient in the tyrosinase activity promoting agent of the present invention, it is also possible to blend in a commercial Japanese pepper extract [Harvex Japanese pepper extract (Kaei Kogyo), Pharcorex Japanese pepper (Ichimaru Pharcos Co. Ltd.), Japanese pepper extract (Maruzen Pharmaceuticals Co., Ltd.), etc.]
[6] The extract thus obtained can be pharmaceutically prepared by using, according to the target formulation, diluting agents, anionic surfactants, non-ionic surfactants, cationic surfactants, ampholytic surfactants, higher alcohols, oils, humectants, thickeners, solvents, usability improvement agents, preservatives, antioxidants, sequestering agents, anti-ultraviolet light agents, powder, other active ingredients, coloring agents, perfume, etc., which are regularly used in compositions for use on hair or endermic liniment compositions (cosmetics or drugs). The aforementioned additives used in the present invention cannot necessarily be distinguished based on a single function, but they will be described according to a common classification.
   Examples of the diluting agents include water, ethanol, isopropyl alcohol and glycols.
   Examples of the anionic surfactants include alkylbenzensulfonate, polyoxyalkylenealkylsulfuric ester salt, alkylsulfuric ester salt, olefinsulfonate, alkylphosphonate, polyoxyalkylenealkyl ether phosphate, dialkylsulfosuccinate and fatty acid salt.
   Examples of the non-ionic surfactants include polyoxyethylenealkyl ether, polyoxyethylene fatty acid ester, polyhydric alcohol fatty acid partial ester, polyoxyethylene polyhydric alcohol fatty acid partial ester, polyglycerine fatty acid ester, polyoxyethylene hydrogenated castor oil derivatives and fatty acid diethanol amine.
   Examples of the cationic surfactants include tertiary aliphatic amine salt, alkyltrimethylammonium halide, dialkyldimethylammonium halide and alkyldimethylbenzylammonium halide.
   Examples of the ampholytic surfactants include the amide betaine type, imidazolium betaine type and sulfobetaine type surfactants.
   Examples of the higher alcohols include cetyl alcohol, stearyl alcohol and behenyl alcohol.
   Examples of the oils include higher fatty acids, solid paraffin, liquid paraffin, silicone oil, polymer silicone and its derivatives, squalane, vaseline and ester oil.
   Examples of the humectant include glycerine, propylene glycol, 1,3-butylene glycol, dipropylene glycol and sorbitol.
   Examples of the thickener include methyl cellulose, hydroxyethyl cellulose, carrageenan, carboxymethyl cellulose and cationized cellulose.
   Example of the powder include silica and resin powder such as nylon powder and polyethylene powder.
   The composition for preventing gray hair of the present invention can contain any ingredient which can be used as an ingredient of a hair restoration agent as long as there is no adverse effect on the effect of the present invention. Such ingredients include active ingredients which are suggested to have their own hair growing effect as well as assistants. Examples include plant extracts such as the Swertia japonica extract and the carrot extract, vitamins such as vitamin B₆, vitamin E and its derivatives, pantothenic acid and its derivatives, glycyrrhizic acid and its derivatives, nicotinic esters such as benzyl nicotinate, amino acids such as serine and methionine, cepharanthine, carpronium chloride, minoxidil, nicorandil, acetylcholine derivatives, cyclosporins and female sex hormone drugs such as estradiol as well as mixtures thereof.
   Furthermore, antimicrobial agents including hinokitiol, hexachlorophene, benzalkonium chloride, cetylpyridinium chloride, undecylenic acid, trichlorocarbanilide and bithionol, refreshing agents such as menthol, salicylic acid, zinc and its derivatives and lactic acid and its alkyl esters, which are active substances, as well as organic acids such as citric acid and amino acids such as arginine can be added to the composition of the present invention.
   The blend ratio of the plant extract, in a dray form, of the present invention should be 0.001-10 wt%, preferably 0.01-1 wt%, of the total weight of the composition. If it is less than 0.001 wt%, then the object of the present invention may not be fully achieved. On the other hand, if it is more than 10 wt%, then pharmaceutical preparation may become difficult or the cost may become a problem.
   The composition for use on the scalp of the present invention refers to a wide range of compositions which are used on the scalp or the hair on the head. Examples include a shampoo, rinse, hair tonic, hair conditioner, a hair restoration agent such as a scalp treatment agent, as well as a hair dressing such as hair liquid, hair spray and hair cream.
   The composition of the present invention can take any form which allows application on the scalp or the skin such as liquid, emulsion and ointment. Generally, the product form can be an emollient lotion, emollient cream, moisture gel, toilet water, hair tonic, hair liquid, scalp treatment, hair cream, aerozol mousse, aerozol spray, hair gel, spray mousse, etc.

### EXAMPLES

The present invention is described in detail below by referring to examples. The present invention is not limited to these examples. The blend ratios are in weight percent units. Before explaining the examples, the extraction method and the evaluation method of the plant extracts used in the present invention are described.

### [1] "Examples for the inventions of claim 1 and claim 2"

### (1) Extraction method

The plant extracts were prepared using the parts and the extraction method shown in the following Table 1. As for the extraction method, 500 g of the crude drug was immersed in 100% methanol. The extraction times are indicated in the table. The obtained plant extracts were used to prepare the compositions for use on the scalp described later.

**Table 1**

| Plant | Part | Extraction time | Yield (%) |
|---|---|---|---|
| Kayu rapet | Bark | 8 days | 2.4 |
| Kemukus | Fruits | 8 days | 1.2 |
| Tempuyung | Leaves | 4 days | 11.1 |
| Belantas | Leaves | 6 days | 5.0 |
| Mesoyi | Bark | 8 days | 0.8 |
| Pule | Branches | 9 days | 2.2 |
| Pulowaras | Bark | 4 days | 2.0 |
| Sintok | Whole plant | 8 days | 3.02 |

### (2) Actual use testing

Fifty men and fifty women, age 18-35, who had troubles with dandruff used the hair tonic after washing their hair everyday and the symptoms were evaluated after two weeks. For the shampoos, the subjects shampooed everyday and the symptoms were evaluated after two weeks in the same manner.

### (Evaluation)

The preventive and/or curing effect with regard to dandruff through actual use was evaluated using the following four grades. The ratio of the "effective" or better in all the cases was defined as the efficacy ratio and shown in tables.
Very effective: A significant reduction in dandruff and the disappearance of itching was observed.
Effective: A reduction in dandruff and alleviation of itching was observed.
Somewhat effective: A reduction in dandruff or alleviation of itching was observed.
Aggravated: Increased dandruff and aggravated itching were observed after use.

### Examples 1-4, Comparative examples 1

Hair tonics were prepared according to the recipes listed in Table 2, and the preventive and/or curing effect with regard to dandruff was evaluated using the aforementioned method. The results are also shown in Table 2.

**Table 2**

| | Examples | | | | Comparative examples |
|---|---|---|---|---|---|
| | 1 | 2 | 3 | 4 | 1 |
| Glycerine | 10.0 | 10.0 | 10.0 | 10.0 | 10.0 |
| 1,3-butylene glycol | 4.0 | 4.0 | 4.0 | 4.0 | 4.0 |
| Ethanol | 7.0 | 7.0 | 7.0 | 7.0 | 7.0 |
| Polyoxyethylene (20 moles) oleyl alcohol ether | 0.5 | 0.5 | 0.5 | 0.5 | 0.5 |
| Kayu rapet bark extract | 1.0 | - | - | - | - |
| Kemukus fruit extract | - | 1.0 | - | - | - |
| Tempuyung leave extract | - | - | 1.0 | - | - |
| Belantas leave extract | - | - | - | 1.0 | - |
| Piroctone olamine | - | - | - | - | 1.0 |
| Purified water | Balance | Balance | Balance | Balance | Balance |
| Efficacy ratio (%) | 74 | 72 | 80 | 80 | 65 |

### Examples 5-8, Comparative example 2

Shampoos were prepared according to the recipes listed in Table 3, and the preventive and/or curing effect with regard to dandruff was evaluated. The results are also shown in Table 3.

**Table 3**

| | Examples | | | | Comparative examples |
|---|---|---|---|---|---|
| | 5 | 6 | 7 | 8 | 2 |
| Sodium cocoylmethyltaurate | 1.0 | 1.0 | 1.0 | 1.0 | 1.0 |
| Lauric acid diethanolamide | 4.0 | 4.0 | 4.0 | 4.0 | 4.0 |
| Ethylene glycol fatty acid ester | 1.5 | 1.5 | 1.5 | 1.5 | 1.5 |
| Propylene glycol | 2.0 | 2.0 | 2.0 | 2.0 | 2.0 |
| Disodium edetate | 0.1 | 0.1 | 0.1 | 0.1 | 0.1 |
| Betaine lauryldimethylamino acetate | 8.0 | 8.0 | 8.0 | 8.0 | 8.0 |
| Mesoyi bark extract | 1.0 | - | - | - | - |
| Pule branch extract | - | 1.0 | - | - | - |
| Pulowaras bark extract | - | - | 1.0 | - | - |
| Sintok whole plant extract | - | - | - | 1.0 | - |
| ZPT | - | - | - | - | 1.0 |
| Perfume | Appropriate amount | Appropriate amount | Appropriate amount | Appropriate amount | Appropriate amount |
| Purified water | Balance | Balance | Balance | Balance | Balance |
| Efficacy ratio (%) | 73 | 75 | 78 | 78 | 66 |

As clearly shown in Table 3, the composition for use on the scalp of the present invention exhibited a superior effect in preventing dandruff generation, curing dandruff and alleviating itching.

### Example 9 Hair tonic

| | |
|---|---|
| (1) Kayu rapet bark extract | 1.0 wt% |
| (2) Lauryldimethylamine oxide | 0.5 |
| (3) Sodium laurylsulfate | 0.05 |
| (4) Propylene glycol | 5.0 |
| (5) Sodium hyaluronate | 0.01 |
| (6) Perfume | Appropriate amount |
| (7) 75% ethanol | Balance |

### Example 10 Rinse

| | |
|---|---|
| (1) Kemukus fruit extract | 0.3 wt% |
| (2) Stearyltrimetylammonium chloride | 2.0 |
| (3) Cetyl alcohol | 2.0 |
| (4) Silicone oil | 3.0 |
| (5) Polyethylene (10 moles) oleyl alcohol ether | 1.0 |
| (6) Glycerine | 5.0 |
| (7) Preservative | Appropriate amount |
| (8) Perfume | Appropriate amount |
| (9) Purified water | Balance |

### Example 11 Emulsion for the scalp

| | |
|---|---|
| (1) Tempuyung leave extract | 5.0 wt% |
| (2) Stearic acid | 1.5 |
| (3) Cetyl alcohol | 0.5 |
| (4) Beeswax | 2.0 |
| (5) Polyoxyethylene (10 moles) oleyl alcohol ether | 1.0 |
| (6) Propylene glycol | 5.0 |
| (7) Ethanol | 3.0 |
| (8) Preservative | Appropriate amount |
| (9) Perfume | Appropriate amount |
| (10) Purified water | Balance |

### Example 12 Hair tonic

| | |
|---|---|
| (1) Belantas leave extract | 0.001 wt% |
| (2) Mesoyi bark extract | 0.001 |
| (3) Polyoxyethylene (8 moles) oleyl alcohol ether | 0.05 |
| (4) 1,3-butylene glycol | 0.2 |
| (5) Perfume | Appropriate amount |
| (6) 60% ethanol | Balance |

### Example 13 Shampoo

| | |
|---|---|
| (1) Pulowaras bark extract | 3.5 wt% |
| (2) Pule branch extract | 3.5 |
| (3) Sodium cocoylmethyltaurate | 1.0 |
| (4) Lauric acid diethanolamide | 4.5 |
| (5) Ethylene glycol fatty acid ester | 2.0 |
| (6) Propylene glycol | 3.5 |
| (7) Disodium edetate | 0.1 |
| (8) Betaine lauryldimethylaminoacetate | 5.0 |
| (9) Perfume | Appropriate amount |
| (10) Purified | Balance |

### Example 14 Emulsion for the scalp

| | |
|---|---|
| (1) Sintok whole plant extract | 5.0 wt% |
| (2) Stearic acid | 1.5 |
| (3) Cetyl alcohol | 0.8 |
| (4) Beeswax | 2.5 |
| (5) Polyoxyethylene (10 moles) oleyl alcohol ether | 1.0 |
| (6) Propylene glycol | 7.0 |
| (7) Ethanol | 4.5 |
| (8) Preservative | Appropriate amount |
| (9) Perfume | Appropriate amount |
| (10) Purified water | Balance |

### [2] "Examples for the inventions of claims 3 through 8"

### I. Sample preparation (preparation of the Kayu rapet extract)

50 g of the whole plant (dried) of Parameria laevigata (Kayu rapet) was immersed in 7.5 L of ethanol at room temperature for a week. The solvent was distilled away from the extract solution to obtain 11.9 g of an extract (sample 1).

### II. Testing to verify the effect

Sample 1 was dissolved in dimethylsulfoxide (DMSO) to obtain a 1% solution, and this solution was diluted to adjust the concentration for the following experiments.

### (1) Cell culture method

B16 melanoma culture cells from mice were used. A culture was conducted in a CO₂ incubator (95% air and 5% CO₂) at 37°C using the Eagle MEM medium containing 3% fetus bovine serum (FBS). After 24 hours of culturing, the sample solution was added to it such that the final concentration (in dried extract) was 10⁻³-10⁻⁵ wt%. The culture was continued for 3 more days, following which time melanin production was visually evaluated and the tyrosinase activity inhibition effect was measured with the following methods.

### (2) Visual evaluation of the amount of melanin

A diffusion plate was placed on top of the lid of the well plate and the amount of melanin in the cells was evaluated using an inverted microscope. For the control, a sample with no added plant extract (control 1) was used. The following criteria were used for the evaluation. The results are shown in Table 1.

### 〈Judgment criteria〉

+: Blacker than control 1 (more melanin)
+/-: Somewhat blacker than control 1 (somewhat more melanin)
-: Almost same as control 1 (almost no melanin was observed.)

### (3) Tyrosinase activity measurement

The medium in the well was removed, followed by washing twice with 100 microliters of PBS. 45 microliters of PBS containing 1% Triton X (surfactant from Rohm & Haas) was then added to each well. The plate was vibrated for 1 minute to thoroughly destroy the cell membranes, and the absorbance at 475 nm was measured using a microplate reader, which was defined as the absorbance at time 0 minutes. Immediately following, 5 microliters of 10 mM L-Dopa solution was added and the plate was transferred to an incubator kept at 37°C to react for 60 minutes.

The plate was vibrated for 1 minute and the absorbance (475 nm) at time 60 minutes was measured. An increase in the absorbance difference between time 0 minutes and time 60 minutes, after the addition of dopa, for the sample to which the plant extract was added compared with this absorbance difference for the sample to which the plant extract was not added (control 1) was obtained. The results are shown in Table 4.

**Table 4**

| Test | Visual evaluation of melanin production | | | Tyrosinase activity promotion ratio (%) | | |
|---|---|---|---|---|---|---|
| Concentration (wt%) | 10⁻⁵ | 10⁻⁴ | 10⁻³ | 10⁻⁵ | 10⁻⁴ | 10⁻³ |
| Control 1 | - | - | - | 3 | 5 | 6 |
| Sample 1 | + | + | + | 28 | 43 | 56 |

### (4) Human gray hair prevention effect by cumulative application

Forty male and female testees at the age of 40-60 with gray hair used the lotion of the present invention and the control lotion, which was the lotion of the present invention without sample 1, on the left and right sides of their scalps, respectively, i.e. the "half head method" twice a day (morning and evening) for four months consecutively. The conditions of the applied sites before and after the testing were compared and the gray hair prevention/alleviation effect was evaluated using the following testing method and judgment criteria.

### (Lotion which was used)

### Lotion of the present invention

| Ingredients | Blend ratio |
|---|---|
| (1) Kayu rapet extract (sample 1) (dried) | 0.5 |
| (2) Polyvinylpyrrolidone/vinyl acetate copolymer | 5.0 |
| (3) Ethyl alcohol | 30.0 |
| (4) Silicone derivative | 0.5 |
| (5) Glycerine | 2.0 |
| (6) Preservative | Appropriate amount |
| (7) Perfume | Appropriate amount |
| (8) Purified water | Balance |

### (Preparation method)

(2), (6) and (7) were added to (3) and homogeneously dissolved. The water phase ((8), (4) and (5), dissolved in advance) was added to this and dissolved.

### Control lotion

Preparation was carried out in the same manner as for the lotion of the present invention as described above except for the fact that (1) was excluded.

### (Test method)

The lotion of the present invention and the control lotion were applied by the testees on the left and right sides of their scalps, respectively, i.e. the "half head method", every day for four months. The number of gray hairs in 1,000 hairs on top of the head was counted before the beginning of the application and four months after beginning the application.

### 〈Judgment criteria〉

++ (very effective): For a half (20) or more of the testees, the number of gray hairs after the application was less than 80% of that before beginning the application.
+: (effective): For a half (20) or more of the testees, the number of gray hairs after the application was 80% or more and less than 90% of that before beginning the application.
+/-: (somewhat effective): For a half (20) or more of the testees, the number of gray hair after the application was 90% or more and less than 100% of that before beginning the application.
-: (not effective): For less than a half (20) the testees, the number of gray hair after the application was 90% or more and less than 100% of that before beginning the application.

The results are shown in Table 5.

**Table 5**

| Sample substance | Gray hair prevention effect |
|---|---|
| Control lotion | - |
| Lotion of the present invention | + |

### III. Examples (pharmaceutical preparation example)

### (Example 1) Hair tonic

| Ingredients | Blend ratio |
|---|---|
| (1) Hydrogenated castor oil EO (40 moles) adduct | 2.0 |
| (2) Kayu rapet extract (sample 1) (dried) | 0.1 |
| (3) 95% ethanol | 70.0 |
| (4) Perfume | Appropriate amount |
| (5) Purified water | Balance |

### (Preparation method)

(1), (2) and (4) were added to (3) and, after stirring and dissolving the mixture, (5) was added to obtain the title composition (product).

### (Example 2) Hair tonic

| Ingredients | Blend ratio |
|---|---|
| (1) Glycerine | 2.0 |
| (2) L-Menthol | 0.1 |
| (3) Kayu rapet extract (sample 1) (dried) | 0.5 |
| (4) 95% ethanol | 60.0 |
| (5) Perfume | Appropriate amount |
| (6) Purified water | Balance |

### (Preparation method)

(1), (2), (3) and (5) were added to (4) and, after stirring and dissolving the mixture, (6) was added to obtain the title composition (product).

### (Example 3) Hair tonic

| Ingredients | Blend ratio |
|---|---|
| (1) Polyethylene glycol 200 | 2.0 |
| (2) L-Menthol | 0.2 |
| (3) Kayu rapet extract (sample 1) (dried) | 1.0 |
| (4) 95% ethanol | 50.0 |
| (5) Perfume | Appropriate amount |
| (6) Purified water | Balance |

### (Preparation method)

(1), (2), (3) and (5) were added to (4) and, after stirring and dissolving the mixture, (6) was added to obtain the title composition (product).

### (Example 4) Hair liquid

| Ingredients | Blend ratio |
|---|---|
| (1) Polyoxypropylbutyl ether (40PO) | 15.0 |
| (2) Polyoxypropylbutyl ether phosphoric acid (40PO) | 15.0 |
| (3) Kayu rapet extract (sample 1) (dried) | 0.001 |
| (4) 1,3-butylene glycol | 5.0 |
| (5) 95% ethanol | 50.0 |
| (6) Perfume | Appropriate amount |
| (7) Pigment | Appropriate amount |
| (8) Edetic acid | Appropriate amount |
| (9) Purified water | Balance |

### (Preparation method)

(1)-(4) and (6)-(8) were added to (5) and, after stirring and dissolving the mixture, (9) was added to obtain the title composition (product).

### (Example 5) Scalp treatment

| Ingredients | Blend ratio |
|---|---|
| (1) 1,3-propylene glycol | 0.5 |
| (2) Pentaerythritoltetra-2-ethylhexanoate | 1.2 |
| (3) 95% ethanol | 60.0 |
| (4) Kayu rapet extract (sample 1) (dried) | 0.00001 |
| (5) Swertia japonica extract | 1.0 |
| (6) Perfume | Appropriate amount |
| (7) DME/LPG (95/5) | Balance |

### (Preparation method)

(1), (2) and (4)-(6) were added to (3) and, after stirring and dissolving the mixture, (7) was added to obtain the title composition (product).

### (Example 6) Hair cream

| Ingredients | Blend ratio |
|---|---|
| | |

| (A phase) | |
|---|---|
| (1) Liquid paraffin | 5.0 |
| (2) Cetostearyl alcohol | 5.5 |
| (3) Vaseline | 5.5 |
| (4) Glyceryl monostearate | 3.0 |
| (5) EO (20 moles added)-2-octyldodecyl ether | 3.0 |
| (6) Vitamin E acetate | 0.05 |
| (7) Propyl paraben | 0.3 |
| (8) Perfume | 0.05 |

| (B phase) | |
|---|---|
| (9) Kayu rapet extract (sample 1) (dried) | 0.001 |
| (10) Glycerine | 7.0 |
| (11) Dipropylene glycol | 20.0 |
| (12) Polyoxyethylens glycol 4000 | 5.0 |
| (13) Sodium hexamethaphosphate | 0.005 |
| (14) Purified water | Balance |

### (Preparation method)

The A phase was heated/dissolved and mixed, to which a heated/dissolved mixture of the B phase was added, and, after emulsification with a homogenizer, the title composition (product) was obtained.

### (Example 7) Hair gel

| Ingredients | Blend ratio |
|---|---|
| (1) Carboxyvinyl polymer | 0.7 |
| (2) Polyvinylpyrrolidone | 2.0 |
| (3) Glycerine | 4.0 |
| (4) Kayu rapet extract (sample 1) (dried) | 0.05 |
| (5) Sodium hydroxide | Appropriate amount |
| (6) Ethyl alcohol | 20.0 |
| (7) Polyoxyethyleneoctyldodecyl ether | Appropriate amount |
| (8) Perfume | Appropriate amount |
| (9) Chelating agent (EDTA) | Appropriate amount |
| (10) Purified water | Balance |

### (Preparation method)

(1) was dispersed using (3) and a part of (10). (2) and (4)-(9) were dissolved in the rest of (10) and then added to the former to while being stirred to obtain the title composition (product).

### (Example 8) Water grease

| Ingredients | Blend ratio |
|---|---|
| (1) Carboxyvinyl polymer | 0.5 |
| (2) Glycerine | 50.0 |
| (3) Sodium hydroxide | Appropriate amount |
| (4) Ethyl alcohol | 10.0 |
| (5) Kayu rapet extract (sample 1) (dried) | 0.0001 |
| (6) Polyoxyethyleneoctyldodecyl ether | Appropriate amount |
| (7) Perfume | Appropriate amount |
| (8) Chelating agent (EDTA) | Appropriate amount |
| (9) Purified water | Balance |

### (Preparation method)

(1) and (3)-(8) were added to (2) and, after stirring and dissolving the mixture, (9) was added to obtain the title composition (product).

### (Example 9) Hair spray

| Ingredients | Blend ratio |
|---|---|
| | |

| (Mother solution recipe) | |
|---|---|
| (1) Acrylic resin arcanol amine solution (50%) | 7.0 |
| (2) Cetyl alcohol | 0.1 |
| (3) Silicone oil (methylphenyl polysiloxane) | 0.3 |
| (4) Ethyl alcohol | 92.6 |
| (5) Kayu rapet extract (sample 1) (dried) | 0.005 |
| (6) Perfume | Appropriate amount |

| (Filling recipe) | |
|---|---|
| (7) Mother solution ((1)-(6)) | 50.0 |
| (8) Dimethyl ether | 45.0 |
| (9) LPG | 5.0 |

### (Preparation method)

(1)-(3) were homogeneously emulsified by a homogenizer. This mixture was then added to a solution of (4)-(6) to prepare a mother solution (7). As for the filling process, a can was filled with (7) and (8), a valve was attached to it, and then it was filled with (9) to obtain the title composition (product).

### (Example 10) Toilet water

| Ingredients | Blend ratio |
|---|---|
| (1) 1,3-butylene glycol | 6.0 |
| (2) Glycerine | 4.0 |
| (3) Oleyl alcohol | 0.1 |
| (4) POE (20) sorbitan monolauric ester | 0.5 |
| (5) POE (15) lauryl alcohol ether | 0.5 |
| (6) Kayu rapet extract (sample 1) (dried) | 1.0 |
| (7) Ethanol | 10.0 |
| (8) Perfume | Appropriate amount |
| (9) Pigment | Appropriate amount |
| (10) Preservative | Appropriate amount |
| (11) Anti-fading agent | Appropriate amount |
| (12) Buffer | Appropriate amount |
| (13) Purified water | Balance |

### (Preparation method)

(1), (2), (11) and (12) were dissolved in (13) at room temperature to obtain the water phase. (3)-(6), (8) and (10) were dissolved in (7) and this mixture was mixed and solubilized in the water phase. (9) was then used to adjust the color to obtain the title composition (product).

### (Example 11) Emollient lotion

| Ingredients | Blend ratio |
|---|---|
| (1) Stearic acid | 2.0 |
| (2) Cetyl alcohol | 1.5 |
| (3) Vaseline | 4.0 |
| (4) Squalane | 5.0 |
| (5) Kayu rapet extract (sample 1) (dried) | 0.0001 |
| (6) Glycerol tri-2-ethylhexanoic ester | 2.0 |
| (7) Sorbitan monooleic ester | 2.0 |
| (8) Dipropylene glycol | 5.0 |
| (9) Polyethylene glycol 1500 | 3.0 |
| (10) Triethanol amine | 1.0 |
| (11) Perfume | Appropriate amount |
| (12) Preservative | Appropriate amount |
| (13) Purified water | Balance |

### (Preparation method)

Preparation was conducted according to a conventional method.

### (Example 12) Emollient cream

| Ingredients | Blend ratio |
|---|---|
| (1) Cetyl alcohol | 5.0 |
| (2) Stearic acid | 3.0 |
| (3) Vaseline | 5.0 |
| (4) Squalane | 10.0 |
| (5) Glycerol tri-2-ethylhexanoic ester | 7.0 |
| (6) Kayu rapet extract (sample 1) (dried) | 0.005 |
| (7) Dipropylene glycol | 5.0 |
| (8) Glycerine | 5.0 |
| (9) Propylene glycol monostearic ester | 8.0 |
| (10) Polyoxyethylene (20) cetyl alcohol ether | 3.0 |
| (11) Triethanol amine | 1.0 |
| (12) Perfume | Appropriate amount |
| (13) Preservative | Appropriate amount |
| (14) Antioxidant | Appropriate amount |
| (15) Purified water | Balance |

### (Preparation method)

Preparation was conducted according to a conventional method.

### (Example 13) Moisture gel

| Ingredients | Blend ratio |
|---|---|
| (1) Dipropylene glycol | 7.0 |
| (2) Polyethylene glycol 1500 | 8.0 |
| (3) Kayu rapet extract (sample 1) (dried) | 0.001 |
| (4) Carboxyvinyl polymer | 0.4 |
| (5) Methyl cellulose | 0.2 |
| (6) Polyoxyethylene (15) oleyl alcohol ether | 1.0 |
| (7) Potassium hydroxide | 0.1 |
| (8) Perfume | Appropriate amount |
| (9) Pigment | Appropriate amount |
| (10) Preservative | Appropriate amount |
| (11) Anti-fading agent | Appropriate amount |
| (12) Chelating agent | Appropriate amount |
| (13) Purified water | Balance |

### (Preparation method)

Preparation was conducted according to a conventional method.

### [3] "Examples for the inventions of claims 9 through 14"

### Example 1: Preparation of the extract

50 g of the whole plant (dried) of Pinguica was immersed in 7.5 L of ethanol at room temperature for a week. The solvent was distilled away from the extract solution to obtain 4.59 g of an extract (Example 1).

Ethanol extraction, in the same manner as in Example 1, was separately conducted for 50 g of the whole plant (dried) of Aritaso (in Japanese; scientific name: Chenopodium Ambrosioides L.), 50 g of the whole plant (dried) of Zapote and 50 g of the whole plant (dried) of Axcopaque to obtain 3.76 g (Example 2), 6.23 g (Example 3) and 4.78 g (Example 4), respectively, of these extracts.

### (Testing to verify the effect)

Each of the extracts obtained in the aforementioned manner was dissolved in dimethylsulfoxide (DMSO) to obtain a 1% solution, and this solution was diluted to adjust the concentration for the following experiments.

### (1) Cell culture method

B16 melanoma culture cells from mice were used. A culture was conducted in a CO₂ incubator (95% air and 5% CO₂) at 37°C using the Eagle MEM medium containing 3% fetus bovine serum (FBS). After 24 hours of culturing, the sample solution was added to it such that the final concentration (in dried extract) was 10⁻³-10⁻⁵ wt%. The culture was continued for 3 more days, following which time melanin production was visually evaluated and the tyrosinase activity inhibition effect was measured with the following methods.

### (2) Visual evaluation of the amount of melanin

A diffusion plate was placed on top of the lid of the well plate and the amount of melanin in the cells was evaluated using an inverted microscope and then a comparison was made with a sample with no added plant extract (the control). The results are shown in Table 6.

### 〈Judgment criteria〉

+: Blacker (more melanin)
+/-: Somewhat blacker (somewhat more melanin)
-: Control (almost no melanin was observed.)

### (3) Tyrosinase activity measurement

The medium in the well was removed, followed by washing twice with 100 microliters of PBS. 45 microliters of PBS containing 1% Triton X (surfactant from Rohm & Haas) was then added to each well. The plate was vibrated for 1 minute to thoroughly destroy the cell membranes, and the absorbance at 475 nm was measured using a microplate reader, which was defined as the absorbance at time 0 minutes. Immediately following, 5 microliters of 10 mM L-Dopa solution was added and the plate was transferred to an incubator kept at 37°C to react for 60 minutes.

The plate was vibrated for 1 minute and the absorbance (475 nm) at time 60 minutes was measured. An increase in the absorbance difference between time 0 minutes and time 60 minutes, after the addition of dopa, for the sample to which the plant extract was added compared with this absorbance difference for the sample to which the plant extract was not added (control) was obtained.

**Table 6**

| Test | Visual evaluation of melanin production | | | Tyrosinase activity promotion ratio (%) | | |
|---|---|---|---|---|---|---|
| Concentration (wt%) | 10⁻⁴ | 10⁻³ | 10⁻² | 10⁻⁴ | 10⁻³ | 10⁻² |
| Nothing added | - | - | - | 3 | 5 | 6 |
| Pinguica extract | + | + | + | 56 | 56 | 6 |
| Aritaso extract | + | + | + | 48 | 53 | 12 |
| Zapote extract | + | + | + | 37 | 49 | 8 |
| Axcopaque extract | + | + | + | 59 | 41 | 2 |

### (4) Human gray hair prevention effect by cumulative application

### (Testing method)

Forty male and female testees at the age of 40-60 with gray hair used the sample of the present invention and the sample of the control example on the left and right sides of their scalps, respectively, i.e. the "half head method" twice a day (morning and evening) for four months consecutively. The conditions of the applied sites before and after the testing were compared and the gray hair prevention/alleviation effect was evaluated.

The lotion containing the effective ingredient of the present invention was applied every day and the gray hair prevention ratio was determined by counting the number of gray hairs in 1,000 hairs on top of the head before the beginning of the application and four months after beginning the application.

### 〈Judgment criteria〉

++ (very effective): For 50% or more of the testees, the number of gray hairs after the application was less than 80% of that before beginning the application.
+: (effective): For 50% or more of the testees, the number of gray hairs after the application was less than 90% of that before beginning the application.
+/-: (somewhat effective): For 50% or more of the testees, the number of gray hair after the application was less than 100% of that before beginning the application.
-: (not effective): For less than 50% the testees, the number of gray hair after the application was less than 100% of that before beginning the application.

The results are shown in Table 7.

For this testing, various gray hair preventive lotions were prepared by using the following compositions and the gray hair preventive effect of their cumulative application was investigated.

| Ingredients | wt% |
|---|---|
| Subject substance | 1.0 |
| Polyvinylpyrrolidone/vinyl acetate copolymer | 5.0 |
| Preservative | Appropriate amount |
| Perfume | Appropriate amount |
| Ethyl alcohol | 30.0 |
| Purified water | 62.5 |
| Silicone derivative | 0.5 |
| Glycerine | 2.0 |

### (Preparation method)

The polymers, preservative and perfume were added to and homogeneously dissolved in ethyl alcohol.

The water phase (purified water, silicone derivative and glycerine), dissolved beforehand, was added to this mixture.

**Table 7**

| Degree of gray hair prevention when various extracts are added | |
|---|---|
| Subject substance | Degree of gray hair prevention |
| Nothing added | - |
| Pinguica extract | + |
| Aritaso extract | + |
| Zapote extract | + |
| Axcopaque extract | + |

Other formulation examples of the present invention are shown below.

### Formulation example 1: Hair tonic

| | |
|---|---|
| (1) Hydrogenated castor oil EO (40 moles) adduct | 2.0 wt% |
| (2) Pinguica extract (dried) | 0.1 |
| (3) 95% ethanol | 70.0 |
| (4) Perfume | Appropriate amount |
| (5) Purified water | Balance |

### (Preparation method)

(1), (2) and (4) were added to (3) and, after stirring and dissolving the mixture, (5) was added to obtain the title composition (product).

### Formulation example 2: Hair tonic

| | |
|---|---|
| (1) Glycerine | 2.0 wt% |
| (2) L-Menthol | 0.1 |
| (3) Aritaso extract (dried) | 0.5 |
| (4) 95% ethanol | 60.0 |
| (5) Perfume | Appropriate amount |
| (6) Purified water | Balance |

### (Preparation method)

(1), (2), (3) and (5) were added to (4) and, after stirring and dissolving the mixture, (6) was added to obtain the title composition (product).

### Formulation example 3: Hair tonic

| | |
|---|---|
| (1) Polyethylene glycol 200 | 2.0 wt% |
| (2) L-Menthol | 0.2 |
| (3) Zapote extract (dried) | 1.0 |
| (4) 95% ethanol | 50.0 |
| (5) Perfume | Appropriate amount |
| (6) Purified water | Balance |

### (Preparation method)

(1), (2), (3) and (5) were added to (4) and, after stirring and dissolving the mixture, (6) was added to obtain the title composition (product).

### Formulation example 4: Hair liquid

| | |
|---|---|
| (1) Polyoxypropylbutyl ether (40PO) | 15.0 wt% |
| (2) Polyoxypropylbutyl ether phosphoric acid (40PO) | 15.0 |
| (3) Axcopaque extract (dried) | 3.0 |
| (4) 1,3-butylene glycol | 5.0 |
| (5) 95% ethanol | 50.0 |
| (6) Perfume | Appropriate amount |
| (7) Pigment and edetic acid | Appropriate amount |
| (8) Purified water | Balance |

Preparation was carried out in the same manner as in Formulation example 1.

### Formulation example 5: Scalp treatment

| | |
|---|---|
| (1) 1,3-propylene glycol | 0.5 wt% |
| (2) Pentaerythritoltetra-2-ethylhexanoate | 1.2 |
| (3) 95% ethanol | 60.0 |
| (4) Zapote extract (dried) | 2.0 |
| (5) Swertia japonica extract | 1.0 |
| (6) Perfume | Appropriate amount |
| (7) DME/LPG (95/5) | Balance |

Preparation was carried out in the same manner as in Formulation example 1.

### Formulation example 6: Hair cream

| (A phase) | |
|---|---|
| (1) Liquid paraffin | 5.0 wt% |
| (2) Cetostearyl alcohol | 5.5 |
| (3) Vaseline | 5.5 |
| (4) Glyceryl monostearate | 3.0 |
| (5) EO (20 moles added)-2-octyldodecyl ether | 3.0 |
| (6) Vitamin E acetate | 0.05 |
| (7) Propyl paraben | 0.3 |
| (8) Perfume | 0.05 |

| (B phase) | |
|---|---|
| (9) Pinguica extract (dried) | 0.01 |
| (10) Glycerine | 7.0 |
| (11) Dipropylene glycol | 20.0 |
| (12) Polyoxyethylene glycol 4000 | 5.0 |
| (13) Sodium hexamethaphosphate | 0.005 |
| (14) Purified water | Balance |

### (Preparation method)

The A phase was heated/dissolved and mixed, to which a heated/dissolved mixture of the B phase was added, and, after emulsification with a homogenizer, the title composition (product) was obtained.

### Formulation example 7: Hair gel

| | |
|---|---|
| (1) Carboxyvinyl polymer | 0.7 wt% |
| (2) Polyvinylpyrrolidone | 2.0 |
| (3) Glycerine | 4.0 |
| (4) Pinguica extract (dried) | 0.05 |
| (5) Sodium hydroxide | Appropriate amount |
| (6) Ethyl alcohol | 20.0 |
| (7) Polyoxyethyleneoctyldodecyl ether | Appropriate amount |
| (8) Perfume and Chelating agent (EDTA) | Appropriate amount |
| (9) Purified water | Balance |

### (Preparation method)

Carboxyvinyl polymer was dispersed using glycerine and a part of purified water. The rest of the ingredients were dissolved in the rest of the purified water and then added to the former while being stirred to obtain the title composition (product).

### Formulation example 8: Water grease

| | |
|---|---|
| (1) Carboxyvinyl polymer | 0.5 wt% |
| (2) Glycerine | 50.0 |
| (3) Sodium hydroxide | Appropriate amount |
| (4) Ethyl alcohol | 10.0 |
| (5) Aritaso extract (dried) | 5.0 |
| (6) Polyoxyethyleneoctyldodecyl ether | Appropriate amount |
| (7) Perfume and chelating agent (EDTA) | Appropriate amount |
| (8) Purified water | Balance |

### (Preparation method)

Preparation was carried out in the same manner as in Formulation example 5.

### Formulation example 9: Hair spray

| (Mother solution recipe) | |
|---|---|
| (1) Acrylic resin arcanol amine solution (50%) | 7.0 wt% |
| (2) Cetyl alcohol | 0.1 |
| (3) Silicone oil (methylphenyl polysiloxane) | 0.3 |
| (4) Ethyl alcohol | 92.6 |
| (5) Axcopaque extract (dried) | 4.0 |
| (6) Perfume | Appropriate amount |

| (Filling recipe) | |
|---|---|
| (7) Mother solution | 50.0 |
| (8) Dimethyl ether | 45.0 |
| (9) LPG | 5.0 |

### (Preparation method)

(1), (2) and (3) were homogeneously emulsified by a homogenizer. This mixture was then added to a solution of other ingredients to prepare a mother solution. As for the filling process, a can was filled with the mother solution, a valve was attached to it, and then it was filled with the gas to obtain the title composition (product).

### Formulation example 10: Toilet water

| | |
|---|---|
| (1) 1,3-butylene glycol | 6.0 wt% |
| (2) Glycerine | 4.0 |
| (3) Oleyl alcohol | 0.1 |
| (4) POE (20) sorbitan monolauric ester | 0.5 |
| (5) POE (15) lauryl alcohol ether | 0.5 |
| (6) Pinguica (dried) | 1.0 |
| (7) Ethanol | 10.0 |
| (8) Perfume, pigment, preservative, anti-fading agent and buffer | Appropriate amount of each |
| (9) Purified water | Balance |

### (Preparation method)

(1), (2) and the buffer and the anti-fading agent of (8) were dissolved in (9) at room temperature to obtain the water phase. The perfume of (8) and (3)-(6) were dissolved in (7) and this mixture was mixed and solubilized in the aforementioned water phase. The coloring agent of (8) was then used to adjust the color to obtain the title composition (product).

### Formulation example 11: Emollient lotion

| | |
|---|---|
| (1) Stearic acid | 2.0 |
| (2) Cetyl alcohol | 1.5 |
| (3) Vaseline | 4.0 |
| (4) Squalane | 5.0 |
| (5) Zapote extract (dried) | 5.0 |
| (6) Glycerol tri-2-ethylhexanoic ester | 2.0 |
| (7) Sorbitan monooleic ester | 2.0 |
| (8) Dipropylene glycol | 5.0 |
| (9) PEG 1500 | 3.0 |
| (10) Triethanol amine | 1.0 |
| (11) Perfume and preservative | Appropriate amount of each |
| (12) Purified water | Balance |

### (Preparation method)

Preparation was conducted according to a conventional method.

### Formulation example 12: Emollient cream

| | |
|---|---|
| (1) Cetyl alcohol | 5.0 |
| (2) Stearic acid | 3.0 |
| (3) Vaseline | 5.0 |
| (4) Squalane | 10.0 |
| (5) Glycerol tri-2-ethylhexanoic ester | 7.0 |
| (6) Axcopaque extract (dried) | 0.5 |
| (7) Dipropylene glycol | 5.0 |
| (8) Glycerine | 5.0 |
| (9) Propylene glycol monostearic ester | 8.0 |
| (10) Polyoxyethylene (20) cetyl alcohol ether | 3.0 |
| (11) Triethanol amine | 1.0 |
| (12) Perfume, preservative and antioxidant | Appropriate amount of each |
| (13) Purified water | Balance |

### (Preparation method)

Preparation was conducted according to a conventional method.

### Formulation example 13: Moisture gel

| | |
|---|---|
| (1) Dipropylene glycol | 7.0 |
| (2) PEG 1500 | 8.0 |
| (3) Pinguica (dried) | 0.1 |
| (4) Carboxyvinyl polymer | 0.4 |
| (5) Methyl cellulose | 0.2 |
| (6) POE (15) oleyl alcohol ether | 1.0 |
| (7) Potassium hydroxide | 0.1 |
| (8) Perfume, pigment, preservative, anti-fading agent and chelating agent | Appropriate amount of each |
| (9) Purified water | Balance |

### (Preparation method)

Preparation was conducted according to a conventional method.

### [4] "Examples for the inventions of claims 15 through 17"

### Example 1: Preparation of the extract

50 g of the whole plant (dried) of guaco misto was immersed in 7.5 L of ethanol at room temperature for a week. The solvent was distilled away from the extract solution to obtain 2.905 g of an extract (Example 1).

### (Testing to verify the effect)

Each of the extracts obtained in the aforementioned manner was dissolved in dimethylsulfoxide (DMSO) to obtain a 1% solution, and this solution was diluted to adjust the concentration for the following experiments.

### (1) Cell culture method

B16 melanoma culture cells from mice were used. A culture was conducted in a CO₂ incubator (95% air and 5% CO₂) at 37°C using the Eagle MEM medium containing 3% fetus bovine serum (FBS). After 24 hours of culturing, the sample solution was added to it such that the final concentration (in dried extract) was 10⁻³- 10⁻⁵ wt%. The culture was continued for 3 more days, following which time melanin production was visually evaluated and the tyrosinase activity inhibition effect was measured with the following methods.

### (2) Visual evaluation of the amount of melanin

A diffusion plate was placed on top of the lid of the well plate and the amount of melanin in the cells was evaluated using an inverted microscope and then a comparison was made with a sample with no added plant extract (the control). The results are shown in Table 8.

### 〈Judgment criteria〉

+: Blacker (more melanin)
+/-: Somewhat blacker (somewhat more melanin)
-: Control (almost no melanin was observed.)

### (3) Tyrosinase activity measurement

The medium in the well was removed, followed by washing twice with 100 microliters of PBS. 45 microliters of PBS containing 1% Triton X (surfactant from Rohm & Haas) was then added to each well. The plate was vibrated for 1 minute to thoroughly destroy the cell membranes, and the absorbance at 475 nm was measured using a microplate reader, which was defined as the absorbance at time 0 minutes. Immediately following, 5 microliters of 10 mM L-Dopa solution was added and the plate was transferred to an incubator kept at 37°C to react for 60 minutes.

The plate was vibrated for 1 minute and the absorbance (475 nm) at time 60 minutes was measured. An increase in the absorbance difference between time 0 minutes and time 60 minutes, after the addition of dopa, for the sample to which the plant extract was added compared with this absorbance difference for the sample to which the plant extract was not added (control) was obtained.

**Table 8**

| Test | Visual evaluation of melanin production | | | Tyrosinase activity promotion ratio (%) | | |
|---|---|---|---|---|---|---|
| Concentration (wt%) | 10⁻⁴ | 10⁻³ | 10⁻² | 10⁻⁴ | 10⁻³ | 10⁻² |
| Nothing added | - | - | - | 1 | 2 | 2 |
| Guaco misto extract | - | - | Toxic | 1 | 79 | Toxic |

### (4) Human gray hair prevention effect by cumulative application

### (Testing method)

Forty male and female testees at the age of 40-60 with gray hair used the sample of the present invention and the sample of the control example on the left and right sides of their scalps, respectively, i.e. the "half head method", twice a day (morning and evening) for four months consecutively. The conditions of the applied sites before and after the testing were compared and the gray hair prevention/alleviation effect was evaluated.

The lotion containing the effective ingredient of the present invention was applied every day and the gray hair prevention ratio was determined by counting the number of gray hairs in 1,000 hairs on top of the head before the beginning of the application and four months after beginning the application.

### (Judgment criteria)

++ (very effective): For 50% or more of the testees, the number of gray hairs after the application was less than 80% of that before beginning the application.
+: (effective): For 50% or more of the testees, the number of gray hairs after the application was less than 90% of that before beginning the application.
+/-: (somewhat effective): For 50% or more of the testees, the number of gray hair after the application was less than 100% of that before beginning the application.
-: (not effective): For less than 50% the testees, the number of gray hair after the application was less than 100% of that before beginning the application.
The results are shown in Table 9.

For this testing, various gray hair preventive lotions were prepared by using the following compositions and the gray hair preventive effect of their cumulative application was investigated.

| Ingredients | wt% |
|---|---|
| Subject substance | 1.0 |
| Polyvinylpyrrolidone/vinyl acetate copolymer | 5.0 |
| Preservative | Appropriate amount |
| Perfume | Appropriate amount |
| Ethyl alcohol | 30.0 |
| Purified water | 62.5 |
| Silicone derivative | 0.5 |
| Glycerine | 2.0 |

### (Preparation method)

The polymers, preservative and perfume were added to and homogeneously dissolved in ethyl alcohol.

The water phase (purified water, silicone derivative and glycerine), dissolved beforehand, was added to this mixture.

**Table 9**

| Degree of gray hair prevention when various extracts are added | |
|---|---|
| Subject substance | Degree of gray hair prevention |
| Nothing added | - |
| Guaco misto extract | + |

Other formulation examples of the present invention are shown below.

### Formulation example 1: Hair tonic

| | |
|---|---|
| (1) Hydrogenated castor oil EO (40 moles) adduct | 2.0 wt% |
| (2) Guaco misto extract (dried) | 0.1 |
| (3) 95% ethanol | 70.0 |
| (4) Perfume | Appropriate amount |
| (5) Purified water | Balance |

### (Preparation method)

(1), (2) and (4) were added to (3) and, after stirring and dissolving the mixture, (5) was added to obtain the title composition (product).

### Formulation example 2: Hair tonic

| | |
|---|---|
| 1) Glycerine | 2.0 wt% |
| (2) L-Menthol | 0.1 |
| (3) Guaco misto extract (dried) | 0.5 |
| (4) 95% ethanol | 60.0 |
| (5) Perfume | Appropriate amount |
| (6) Purified water | Balance |

### (Preparation method)

(1), (2), (3) and (5) were added to (4) and, after stirring and dissolving the mixture, (6) was added to obtain the title composition (product).

### Formulation example 3: Hair tonic

| | |
|---|---|
| (1) Polyethylene glycol 200 | 2.0 wt% |
| (2) L-Menthol | 0.2 |
| (3) Guaco misto extract (dried) | 1.0 |
| (4) 95% ethanol | 50.0 |
| (5) Perfume | Appropriate amount |
| (6) Purified water | Balance |

### (Preparation method)

(1), (2), (3) and (5) were added to (4) and, after stirring and dissolving the mixture, (6) was added to obtain the title composition (product).

### Formulation example 4: Hair liquid

| | |
|---|---|
| (1) Polyoxypropylbutyl ether (40PO) | 15.0 wt% |
| (2) Polyoxypropylbutyl ether phosphoric acid (40PO) | 15.0 |
| (3) Guaco misto extract (dried) | 3.0 |
| (4) 1,3-butylene glycol | 5.0 |
| (5) 95% ethanol | 50.0 |
| (6) Perfume | Appropriate amount |
| (7) Pigment and edetic acid | Appropriate amount |
| (8) Purified water | Balance |

Preparation was carried out in the same manner as in Formulation example 1.

### Formulation example 5: Scalp treatment

| | |
|---|---|
| (1) 1,3-propylene glycol | 0.5 wt% |
| (2) Pentaerythritoltetra-2-ethylhexanoate | 1.2 |
| (3) 95% ethanol | 60.0 |
| (4) Guaco misto extract (dried) | 2.0 |
| (5) Swertia japonica extract | 1.0 |
| (6) Perfume | Appropriate amount |
| (7) DME/LPG (95/5) | Balance |

Preparation was carried out in the same manner as in Formulation example 1.

### Formulation example 6: Hair cream

| (A phase) | |
|---|---|
| (1) Liquid paraffin | 5.0 wt% |
| (2) Cetostearyl alcohol | 5.5 |
| (3) Vaseline | 5.5 |
| (4) Glyceryl monostearate | 3.0 |
| (5) EO (20 moles added)-2-octyldodecyl ether | 3.0 |
| (6) Vitamin E acetate | 0.05 |
| (7) Propyl paraben | 0.3 |
| (8) Perfume | 0.05 |

| (B phase) | |
|---|---|
| (9) Guaco misto extract (dried) | 0.01 |
| (10) Glycerine | 7.0 |
| (11) Dipropylene glycol | 20.0 |
| (12) Polyoxyethylene glycol 4000 | 5.0 |
| (13) Sodium hexamethaphosphate | 0.005 |
| (14) Purified water | Balance |

### (Preparation method)

The A phase was heated/dissolved and mixed, to which a heated/dissolved mixture of the B phase was added, and, after emulsification with a homogenizer, the title composition (product) was obtained.

### Formulation example 7: Hair gel

| | |
|---|---|
| (1) Carboxyvinyl polymer | 0.7 wt% |
| (2) Polyvinylpyrrolidone | 2.0 |
| (3) Glycerine | 4.0 |
| (4) Guaco misto extract (dried) | 0.05 |
| (5) Sodium hydroxide | Appropriate amount |
| (6) Ethyl alcohol | 20.0 |
| (7) Polyoxyethyleneoctyldodecyl ether | Appropriate amount |
| (8) Perfume and chelating agent (EDTA) | Appropriate amount |
| (9) Purified water | Balance |

### (Preparation method)

Carboxyvinyl polymer was dispersed using glycerine and a part of purified water. The rest of the ingredients were dissolved in the rest of the purified water and then added to the former while being stirred to obtain the title composition (product).

### Formulation example 8: Water grease

| | |
|---|---|
| (1) Carboxyvinyl polymer | 0.5 wt% |
| (2) Glycerine | 50.0 |
| (3) Sodium hydroxide | Appropriate amount |
| (4) Ethyl alcohol | 10.0 |
| (5) Guaco misto extract (dried) | 5.0 |
| (6) Polyoxyethyleneoctyldodecyl ether | Appropriate amount |
| (7) Perfume and chelating agent (EDTA) | Appropriate amount |
| (8) Purified water | Balance |

### (Preparation method)

Preparation was carried out in the same manner as in Formulation example 5.

### Formulation example 9: Hair spray

| (Mother solution recipe) | |
|---|---|
| (1) Acrylic resin arcanol amine solution (50%) | 7.0 wt% |
| (2) Cetyl alcohol | 0.1 |
| (3) Silicone oil (methylphenyl polysiloxane) | 0.3 |
| (4) Ethyl alcohol | 92.6 |
| (5) Guaco misto extract (dried) | 4.0 |
| (6) Perfume | Appropriate amount |

| (Filling recipe) | |
|---|---|
| (7) Mother solution | 50.0 |
| (8) Dimethyl ether | 45.0 |
| (9) LPG | 5.0 |

### (Preparation method)

(1), (2) and (3) were homogeneously emulsified by a homogenizer. This mixture was then added to a solution of other ingredients to prepare a mother solution. As for the filling process, a can was filled with the mother solution, a valve was attached to it, and then it was filled with the gas to obtain the title composition (product).

### Formulation example 10: Toilet water

| | |
|---|---|
| (1) 1,3-butylene glycol | 6.0 wt% |
| (2) Glycerine | 4.0 |
| (3) Oleyl alcohol | 0.1 |
| (4) POE (20) sorbitan monolauric ester | 0.5 |
| (5) POE (15) lauryl alcohol ether | 0.5 |
| (6) Guaco misto (dried) | 1.0 |
| (7) Ethanol | 10.0 |
| (8) Perfume, pigment, preservative, anti-fading agent and buffer | Appropriate amount of each |
| (9) Purified water | Balance |

### (Preparation method)

(1) and (2), as well as the buffer and the anti-fading agent of (8) were dissolved in (9) at room temperature to obtain the water phase. The perfume of (8) as well as (3), (4), (5) and (6) were dissolved in (7) and this mixture was mixed and solubilized in the aforementioned water phase. The coloring agent of (8) was then used to adjust the color to obtain the title composition (product).

### Formulation example 11: Emollient lotion

| | |
|---|---|
| (1) Stearic acid | 2.0 |
| (2) Cetyl alcohol | 1.5 |
| (3) Vaseline | 4.0 |
| (4) Squalane | 5.0 |
| (5) Guaco misto extract (dried) | 5.0 |
| (6) Glycerol tri-2-ethylhexanoic ester | 2.0 |
| (7) Sorbitan monooleic ester | 2.0 |
| (8) Dipropylene glycol | 5.0 |
| (9) PEG 1500 | 3.0 |
| (10) Triethanol amine | 1.0 |
| (11) Perfume and preservative | Appropriate amount of each |
| (12) Purified water | Balance |

### (Preparation method)

Preparation was conducted according to a conventional method.

### Formulation example 12: Emollient cream

| | |
|---|---|
| (1) Cetyl alcohol | 5.0 |
| (2) Stearic acid | 3.0 |
| (3) Vaseline | 5.0 |
| (4) Squalane | 10.0 |
| (5) Glycerol tri-2-ethylhexanoic ester | 7.0 |
| (6) Guaco misto extract (dried) | 0.5 |
| (7) Dipropylene glycol | 5.0 |
| (8) Glycerine | 5.0 |
| (9) Propylene glycol monostearic ester | 8.0 |
| (10) Polyoxyethylene (20) cetyl alcohol ether | 3.0 |
| (11) Triethanol amine | 1.0 |
| (12) Perfume, preservative and antioxidant | Appropriate amount of each |
| (13) Purified water | Balance |

### (Preparation method)

Preparation was conducted according to a conventional method.

### Formulation example 13: Moisture gel

| | |
|---|---|
| (1) Dipropylene glycol | 7.0 |
| (2) PEG 1500 | 8.0 |
| (3) Guaco misto (dried) | 0.1 |
| (4) Carboxyvinyl polymer | 0.4 |
| (5) Methyl cellulose | 0.2 |
| (6) POE (15) oleyl alcohol ether | 1.0 |
| (7) Potassium hydroxide | 0.1 |
| (8) Perfume, pigment, preservative, anti-fading agent and chelating agent | Appropriate amount of each |
| (9) Purified water | Balance |

### (Preparation method)

Preparation was conducted according to a conventional method.

### [5] "Examples for the inventions of claims 18 through 20"

### [Preparation example] Preparation of a Japanese pepper extract

500 g of fruit peel of Japanese pepper was immersed in ethanol at room temperature for a week. The solvent was distilled away from the extract solution to obtain 9.7 g of a Japanese pepper extract.

In the following test examples and recipe examples, this extract was used as the "Japanese pepper extract".

### [Test example]

### Testing to verify the effect

The Japanese pepper extract obtained in the manner described above was dissolved in dimethylsulfoxide (DMSO) to obtain a 1% solution, and this solution was diluted to adjust the concentration for the following experiments.

### (1) Cell culture method

B16 melanoma culture cells from mice were used. A culture was conducted in a CO₂ incubator (95% air and 5% CO₂) at 37°C using the Eagle MEM medium containing 3% fetus bovine serum (FBS). After 24 hours of culturing, the sample solution was added to it such that the final concentration (in dried extract) was 10⁻³-10⁻⁵ wt%. The culture was continued for 3 more days, following which time melanin production was visually evaluated and the tyrosinase activity inhibition effect was measured with the following methods.

### (2) Visual evaluation of the amount of melanin

A diffusion plate was placed on top of the lid of the well plate and the amount of melanin in the cells was evaluated using an inverted microscope. A comparison was made with a sample with no added Japanese pepper extract (control). The results are shown in Table 10.

**Table 10**

| | Visual evaluation of melanin production | | |
|---|---|---|---|
| Concentration (wt%) | 10⁻⁵ | 10⁻⁴ | 10⁻³ |
| Not added | - | - | - |
| Japanese pepper extract | - | +/- | + |

### 〈Judgment criteria〉

+: Blacker (amount of melanin)
+/-: Somewhat blacker (amount of melanin)
-: Control (amount of melanin)

It has become clear from these results that when the concentration of the Japanese pepper extract is 10-3 wt% it visibly promotes melanin production.

### (3) Tyrosinase activity measurement

The medium in the well was removed, followed by washing twice with 100 microliters of PBS. 45 microliters of PBS containing 1% Triton X (surfactant from Rohm & Haas) was then added to each well. The plate was vibrated for 1 minute to thoroughly destroy the cell membranes, and the absorbance at 475 nm was measured using a microplate reader, which was defined as the absorbance at time 0 minutes. Immediately following, 5 microliters of 10 mM L-Dopa solution was added and the plate was transferred to an incubator kept at 37°C to react for 60 minutes.

After the reaction, the plate was vibrated for 1 minute and the absorbance (475 nm) at time 60 minutes was measured.

An increase in the absorbance difference between time 0 minutes and time 60 minutes for the sample to which the Japanese pepper extract was added compared with this absorbance difference for the sample to which the Japanese pepper extract was not added (control) was defined as the tyrosinase activity promotion ratio (%). The results are shown in Table 11.

**Table 11**

| | Tyrosinase activity promotion ratio (%) | | |
|---|---|---|---|
| Concentration (wt%) | 10⁻⁵ | 10⁻⁴ | 10⁻³ |
| Not added | 0 | 3 | 6 |
| Japanese pepper extract | 20 | 80 | 90 |

It has become clear from Table 11 that the Japanese pepper extract promotes the tyrosinase activity and is effective in gray hair prevention.

### (4) Human gray hair prevention effect by cumulative application

### 〈Testing method〉

Forty male and female testees at the age of 40-60 with gray hair used the Japanese pepper extract [a lotion which contained these derivatives and a lotion with no added derivative (control)], on the left and right sides of their scalps, respectively, i.e. the "half head method" twice a day (morning and evening) for four months consecutively. The conditions of the applied sites before and after the testing were compared and the gray hair prevention effect was evaluated.

This lotion was applied every day and the gray hair prevention ratio was determined by counting the number of gray hairs in 1,000 hairs on top of the head before the beginning of the application and four months after beginning the application.

### 〈Recipe of the lotion〉

| (Ingredients) | Blend ratio (wt%) |
|---|---|
| Japanese pepper extract | 0.5 |
| Polyvinylpyrrolidone/vinyl acetate copolymer | 5.0 |
| Preservative | Appropriate amount |
| Perfume | Appropriate amount |
| Ethyl alcohol | 30.0 |
| Purified water | 62.5 |
| Silicone derivative | 0.5 |
| Glycerine | 2.0 |

### Preparation method

The Japanese pepper extract, polyvinylpyrrolidone/vinyl acetate copolymer, preservative and perfume were added to and homogeneously dissolved in ethyl alcohol. The water phase (purified water, silicone derivative and glycerine), dissolved beforehand, was added to this mixture and dissolved.

### 〈Judgment criteria〉

### (Judgment criteria)

++ (very effective): For 50% or more of the testees, the number of gray hairs after the application was less than 80% of that before beginning the application.
+: (effective): For 50% or more of the testees, the number of gray hairs after the application was less than 90% of that before beginning the application.
+/-: (somewhat effective): For 50% or more of the testees, the number of gray hair after the application was less than 100% of that before beginning the application.
-: (not effective): For less than 50% the testees, the number of gray hair after the application was less than 100% of that before beginning the application.
The results are shown in Table 12.

**Table 12**

| Sample substance | Gray hair prevention effect |
|---|---|
| Not added | - |
| Japanese pepper extract | + |

It has become clear from this result that the Japanese pepper extract has a gray hair prevention effect.

Other recipes of the tyrosinase activity promoting agent of the present invention are shown below. The aforementioned testing of the human gray hair prevention effect was carried out for the tyrosinase activity promoting agents of the present invention in these examples and every example was evaluated as "effective" or "very effective".

### [Example 1] Hair tonic

| Ingredients | Blend ratio (wt%) |
|---|---|
| (1) Hydrogenated castor oil EO (40 moles) adduct | 2.0 |
| (2) Japanese pepper extract | 1.0 |
| (3) 95% ethanol | 70.0 |
| (4) Perfume | Appropriate amount |
| (5) Purified water | Balance |

### Preparation method

(1), (2) and (4) were added to (3) and, after stirring and dissolving the mixture, (5) was added to obtain the hair tonic.

### [Example 2] Hair tonic

| Ingredients | Blend ratio(wt%) |
|---|---|
| (1) Glycerine | 2.0 |
| (2) L-Menthol | 0.1 |
| (3) Japanese pepper extract | 0.1 |
| (4) 95% ethanol | 60.0 |
| (5) Perfume | Appropriate amount |
| (6) Purified water | Balance |

### Preparation method

(1), (2), (3) and (5) were added to (4) and, after stirring and dissolving the mixture, (6) was added to obtain the hair tonic.

### [Example 3] Hair tonic

| Ingredients | Blend ratio (wt%) |
|---|---|
| (1) Polyethylene glycol 200 | 2.0 |
| (2) L-Menthol | 0.2 |
| (3) Japanese pepper extract | 0.01 |
| (4) 95% ethanol | 50.0 |
| (5) Perfume | Appropriate amount |
| (6) Purified water | Balance |

### Preparation method

(1), (2), (4) and (5) were added to (3) and, after stirring and dissolving the mixture, (6) was added to obtain the hair tonic.

### [Example 4] Hair liquid

| Ingredients | Blend ratio (wt%) |
|---|---|
| (1) Polyoxypropylbutyl ether (40PO) | 15.0 |
| (2) Polyoxypropylbutyl ether phosphoric acid (40PO) | 15.0 |
| (3) Japanese pepper extract | 0.001 |
| (4) 1,3-butylene glycol | 5.0 |
| (5) 95% ethanol | 50.0 |
| (6) Perfume | Appropriate amount |
| (7) Pigment and edetic acid | Appropriate amount |
| (8) Purified water | Balance |

### Preparation method

Preparation was carried out in the same manner as in Example 1.

### [Example 5] Scalp treatment

| Ingredients | Blend ratio (wt%) |
|---|---|
| (1) 1,3-propylene glycol | 0.5 |
| (2) Pentaerythritoltetra-2-ethylhexanoate | 1.2 |
| (3) 95% ethanol | 60.0 |
| (4) Japanese pepper extract | 1.0 |
| (5) Swertia japonica extract | 1.0 |
| (6) Perfume | Appropriate amount |
| (7) DME/LPG (95/5) | Balance |

### Preparation method

Preparation was carried out in the same manner as in Example 1.

### [Example 6] Hair cream

| Ingredients | Blend ratio (wt%) |
|---|---|
| | |

| (A phase) | |
|---|---|
| (1) Liquid paraffin | 5.0 |
| (2) Cetostearyl alcohol | 5.5 |
| (3) Vaseline | 5.5 |
| (4) Glyceryl monostearate | 3.0 |
| (5) EO (20 moles added)-2-octyldodecyl ether | 3.0 |
| (6) Vitamin E acetate | 0.05 |
| (7) Propyl paraben | 0.3 |
| (8) Perfume | 0.05 |

| (B phase) | |
|---|---|
| (9) Japanese pepper extract | 0.5 |
| (10) Glycerine | 7.0 |
| (11) Dipropylene glycol | 20.0 |
| (12) Polyoxyethylene glycol 4000 | 5.0 |
| (13) Sodium hexamethaphosphate | 0.005 |
| (14) Purified water | Balance |

### Preparation method

The A phase was heated/dissolved and mixed, to which a heated/dissolved mixture of the B phase was added, and, after emulsification with a homogenizer, the hair cream was obtained.

### [Example 7] Hair gel

| Ingredients | Blend ratio (wt%) |
|---|---|
| (1) Carboxyvinyl polymer | 0.7 |
| (2) Polyvinylpyrrolidone | 2.0 |
| (3) Glycerine | 4.0 |
| (4) Japanese pepper extract | 0.01 |
| (5) Sodium hydroxide | Appropriate amount |
| (6) Ethyl alcohol | 20.0 |
| (7) Polyoxyethyleneoctyldodecyl ether | Appropriate amount |
| (8) Perfume and edetic acid | Appropriate amount |
| (9) Purified water | Balance |

### Preparation method

(1) was dispersed using (3) and a part of the purified water. Other ingredients were dissolved in the rest of the purified water and then added to the former while being stirred to obtain the hair gel.

### [Example 8] Hair gel

| Ingredients | Blend ratio (wt%) |
|---|---|
| (1) Carboxyvinyl polymer | 0.5 |
| (2) Glycerine | 50.0 |
| (3) Sodium hydroxide | Appropriate amount |
| (4) Ethyl alcohol | 10.0 |
| (5) Japanese pepper extract | 0.001 |
| (6) Polyoxyethyleneoctyldodecyl ether | Appropriate amount |
| (8) Perfume and edetic acid | Appropriate amount |
| (9) Purified water | Balance |

### Preparation method

Preparation was carried out in the same manner as in Example 7.

### [Example 9] Hair spray

| Ingredients | Blend ratio (wt%) |
|---|---|
| | |

| (Mother solution recipe) | |
|---|---|
| (1) Acrylic resin arcanol amine solution (50%) | 7.0 |
| (2) Cetyl alcohol | 0.1 |
| (3) Silicone oil (methylphenyl polysiloxane) | 0.3 |
| (4) Ethyl alcohol | 92.6 |
| (5) Japanese pepper extract | 0.1 |
| (6) Perfume | Appropriate amount |

| (Filling recipe) | |
|---|---|
| (7) Mother solution | 50.0 |
| (8) Dimethyl ether | 45.0 |
| (9) LPG | 5.0 |

### Preparation method

(1), (2), and (3) were homogeneously emulsified by a homogenizer. This mixture was then added to a solution of other ingredients to prepare a mother solution.

As for the filling process, a can was filled with the mother solution, a valve was attached to it, and then it was filled with the gas to obtain the hair spray.

### [6] "Examples for the inventions of claim 10, claim 15, and claim 18: compositions for use on the scalp"

For each extract obtained as described above, the following testing was conducted.

### (1) Actual use testing

Fifty men and fifty women, age 18-35, who had troubles with dandruff used the hair tonic after washing their hair everyday and the symptoms were evaluated after two weeks. For the shampoos, the subjects shampooed everyday and the symptoms were evaluated after two weeks in the same manner.

### (Evaluation)

The preventive and/or curing effect with regard to dandruff through actual use was evaluated using the following four grades. The ratio of the "effective" or better in all the cases was defined as the efficacy ratio and shown in tables.
Very effective: A significant reduction in dandruff and the disappearance of itching was observed.
Effective: A reduction in dandruff and alleviation of itching was observed.
Somewhat effective: A reduction in dandruff or alleviation of itching was observed.
Aggravated: Increased dandruff and aggravated itching were observed after use.

### Examples 1-6, Comparative examples 1

Hair tonics were prepared according to the recipes listed in Table 13, and the preventive and/or curing effect with regard to dandruff was evaluated using the aforementioned method. The results are also shown in Table 13.

**Table 13**

| | Examples | | | | | | Comparative examples |
|---|---|---|---|---|---|---|---|
| | 1 | 2 | 3 | 4 | 5 | 6 | 1 |
| Glycerine | 10.0 | 10.0 | 10.0 | 10.0 | 10.0 | 10.0 | 10.0 |
| 1,3-butylene glycol | 4.0 | 4.0 | 4.0 | 4.0 | 4.0 | 4.0 | 4.0 |
| Ethanol | 7.0 | 7.0 | 7.0 | 7.0 | 7.0 | 7.0 | 7.0 |
| Polyoxyethylene (20 moles) oleyl alcohol ether | 0.5 | 0.5 | 0.5 | 0.5 | 0.5 | 0.5 | 0.5 |
| Pinguica extract | 1.0 | - | - | - | - | - | - |
| Aritaso extract | - | 1.0 | - | - | - | - | - |
| Zapote extract | - | - | 1.0 | - | - | - | - |
| Axcopaque extract | - | - | - | 1.0 | - | - | - |
| Guaco misto | - | - | - | - | 1.0 | - | - |
| Japanese pepper extract | - | - | - | - | - | 1.0 | - |
| Piroctone olamine | - | - | - | - | - | - | 1.0 |
| Purified water | Balance | Balance | Balance | Balance | Balance | Balance | Balance |
| Efficacy ratio (%) | 76 | 74 | 79 | 72 | 75 | 81 | 65 |

### Examples 7-13, Comparative example 2

Shampoos were prepared according to the recipes listed in Table 14, and the preventive and/or curing effect with regard to dandruff was evaluated using the aforementioned method. The results are also shown in Table 14.

**Table 14**

| | Examples | | | | | | Comparative examples |
|---|---|---|---|---|---|---|---|
| | 7 | 8 | 9 | 10 | 11 | 12 | 2 |
| Sodium cocoylmethyltaurate | 1.0 | 1.0 | 1.0 | 1.0 | 1.0 | 1.0 | 1.0 |
| Lauric acid diethanolamide | 4.0 | 4.0 | 4.0 | 4.0 | 4.0 | 4.0 | 4.0 |
| Ethylene glycol fatty acid ester | 1.5 | 1.5 | 1.5 | 1.5 | 1.5 | 1.5 | 1.5 |
| Propylene glycol | 2.0 | 2.0 | 2.0 | 2.0 | 2.0 | 2.0 | 2.0 |
| Disodium edetate | 0.1 | 0.1 | 0.1 | 0.1 | 0.1 | 0.1 | 0.1 |
| Betaine lauryldimethylaminoacetate | 8.0 | 8.0 | 8.0 | 8.0 | 8.0 | 8.0 | 8.0 |
| Pinguica extract | 1.0 | - | - | - | - | - | - |
| Aritaso extract | - | 1.0 | - | - | - | - | - |
| Zapote extract | - | - | 1.0 | - | - | - | - |
| Axcopaque extract | - | - | - | 1.0 | - | - | - |
| Guaco misto | - | - | - | - | 1.0 | - | - |
| Japanese pepper extract | - | - | - | - | - | 1.0 | - |
| ZPT | - | - | - | - | - | - | 1.0 |
| Perfume | Appropriate amount | Appropriate amount | Appropriate amount | Appropriate amont | Appropriate amount | Appropriate amount | Appropriate amount |
| Purified water | Balance | Balance | Balance | Balance | Balance | Balance | Balance |
| Efficacy ratio (%) | 80 | 72 | 75 | 80 | 78 | 83 | 66 |

As clearly shown in Table 14, the composition for use on the scalp of the present invention exhibited a superior effect in preventing dandruff generation, curing dandruff and alleviating itching.

### Example 13 Rinse

| | |
|---|---|
| (1) Pinguica fruit extract | 0.3 wt% |
| (2) Stearyltrimetylammonium chloride | 2.0 |
| (3) Cetyl alcohol | 2.0 |
| (4) Silicone oil | 3.0 |
| (5) Polyethylene (10 moles) oleyl alcohol ether | 1.0 |
| (6) Glycerine | 5.0 |
| (7) Preservative | Appropriate amount |
| (8) Perfume | Appropriate amount |
| (9) Purified water | Balance |

### Example 14 Emulsion for the scalp

| | |
|---|---|
| (1) Aritaso whole plant extract | 5.0 wt% |
| (2) Stearic acid | 1.5 |
| (3) Cetyl alcohol | 0.5 |
| (4) Beeswax | 2.0 |
| (5) Polyoxyethylene (10 moles) oleyl alcohol ether | 1.0 |
| (6) Propylene glycol | 5.0 |
| (7) Ethanol | 3.0 |
| (8) Preservative | Appropriate amount |
| (9) Perfume | Appropriate amount |
| (10) Purified water | Balance |

### Example 15 Hair tonic

| | |
|---|---|
| (1) Japanese pepper fruit extract | 0.001 wt% |
| (2) Zapote leaf extract | 0.001 |
| (3) Polyoxyethylene (8 moles) oleyl alcohol ether | 0.05 |
| (4) 1,3-butylene glycol | 0.2 |
| (5) Perfume | Appropriate amount |
| (6) 60% ethanol | Balance |

### Example 16 Shampoo

| | |
|---|---|
| (1) Axcopaque leaf extract | 3.5 wt% |
| (2) Guaco misto leaf extract | 3.5 |
| (3) Sodium cocoylmethyltaurate | 1.0 |
| (4) Lauric acid diethanolamide | 4.5 |
| (5) Ethylene glycol fatty acid ester | 2.0 |
| (6) Propylene glycol | 3.5 |
| (7) Disodium edetate | 0.1 |
| (8) Betaine lauryldimethylaminoacetate | 5.0 |
| (9) Perfume | Appropriate amount |
| (10) Purified | Balance |

### Example 17 Emulsion for the scalp

| | |
|---|---|
| (1) Japanese pepper fruit extract | 5.0 wt% |
| (2) Stearic acid | 1.5 |
| (3) Cetyl alcohol | 0.8 |
| (4) Beeswax | 2.5 |
| (5) Polyoxyethylene (10 moles) oleyl alcohol ether | 1.0 |
| (6) Propylene glycol | 7.0 |
| (7) Ethanol | 4.5 |
| (8) Preservative | Appropriate amount |
| (9) Perfume | Appropriate amount |
| (10) Purified water | Balance |

### INDUSTRIAL APPLICABILITY OF THE INVENTION

As described thus far, the composition for use on the scalp of the present invention can effectively prevent dandruff and is safe. Also, the composition for use on the scalp of the present invention is particularly effective in preventing and treating dandruff which is generated due to scalp stimulation by surfactants such as shampoos, rinses, etc. It also is highly effective at preventing scalp itching.

Also, the present invention provides a composition with a superior tyrosinase activity promoting action and gray hair prevention action.

## Claims

1. A composition for use on the scalp which characteristically contains one or more types of extracts chosen from among extracts of the following plants.
(1) Kayu rapet (scientific name: Parameria laevigata)
(2) Kemukus (scientific name: Piper cubeba)
(3) Tempuyung (scientific name: Sonchus arvensis L.)
(4) Belantas (scientific name: Pluchea indica L.)
(5) Mesoyi (scientific name: Massoia aromatica Becc.)
(6) Pule (scientific name: Alstonia scholaris)
(7) Pulowaras (scientific name: Alycia reindwartii BI.)
(8) Sintok (scientific name: Cinnamomum sintoc BI.)

2. The composition for use on the scalp of claim 1 wherein the blend ratio of the plant extract is 0.001-10.0 wt%.

3. A composition for use on the scalp which contains as an effective ingredient an extract from a plant of the Apocynaceae family, genus Parameria.

4. The composition for use on the scalp of claim 3 wherein the plant of genus Parameria is Parameria laevigata (Kayu rapet).

5. A composition for promoting tyrosinase activity which contains as an effective ingredient an extract from a plant of the Apocynaceae family, genus Parameria.

6. The composition for promoting tyrosinase activity of claim 5 wherein the plant of genus Parameria is Parameria laevigata (Kayu rapet).

7. A composition for preventing gray hair which contains as an effective ingredient an extract from a plant of the Apocynaceae family, genus Parameria.

8. The composition for preventing gray hair of claim 7 wherein the plant of genus Parameria is Parameria laevigata (Kayu rapet).

9. A composition for use of the scalp which contains as an effective ingredient a plant extract wherein the effective ingredient is at least one type of extract derived from a plant of a genus chosen from a group consisting of the Ericaceae family, genus Arctostaphylos, the Chenopodiaceae family, genus Chenopodium and genus Poterium and the Ericaceae family, genus Gautheria.

10. The composition for use of the scalp of claim 9 wherein the plant of the Ericaceae family, genus Arctostaphylos, is Pinguica (scientific name: Chenopodium Ambrosioides L.); the plant of the Chenopodiaceae family, genus Chenopodium, is Aritaso (in Japanese; scientific name: Chenopodium Ambrosioides L.); the plant of genus Poterium is Zapote (scientific name: Poterium Zapote); and the plant of the Ericaceae family, genus Gautheria, is Axcopaque (scientific name: Gautheria Acuminata).

11. A composition for promoting tyrosinase activity which contains as an effective ingredient a plant extract wherein the effective ingredient is at least one type of extract derived from a plant of a genus chosen from a group consisting of the Ericaceae family, genus Arctostaphylos, the Chenopodiaceae family, genus Chenopodium and genus Poterium and the Ericaceae family, genus Gautheria.

12. A composition for preventing gray hair which contains as an effective ingredient a plant extract wherein the effective ingredient is at least one type of extract derived from a plant of a genus chosen from a group consisting of the Ericaceae family, genus Arctostaphylos, the Chenopodiaceae family, genus Chenopodium and genus Poterium and the Ericaceae family, genus Gautheria.

13. The composition of claim 11 wherein the plant of the Ericaceae family, genus Arctostaphylos, is Pinguica (scientific name: Chenopodium Ambrosioides L.); the plant of the Chenopodiaceae family, genus Chenopodium, is Aritaso (in Japanese; scientific name: Chenopodium Ambrosioides L.); the plant of genus Poterium is Zapote (scientific name: Poterium Zapote); and the plant of the Ericaceae family, genus Gautheria, is Axcopaque (scientific name: Gautheria Acuminata).

14. The composition of claim 12 wherein the plant of the Ericaceae family, genus Arctostaphylos, is Pinguica (scientific name: Chenopodium Ambrosioides L.); the plant of the Chenopodiaceae family, genus Chenopodium, is Aritaso (in Japanese; scientific name: Chenopodium Ambrosioides L.); the plant of genus Poterium is Zapote (scientific name: Poterium Zapote); and the plant of the Ericaceae family, genus Gautheria, is Axcopaque (scientific name: Gautheria Acuminata).

15. A composition for use on the scalp which characteristically contains as an effective ingredient an extract from guaco misto (scientific name: Mykania Glomerata) of the Compositae family, genus Mykania.

16. A composition for promoting tyrosinase activity which contains as an effective ingredient a plant extract wherein the effective ingredient is an extract from guaco misto (scientific name: Mykania Glomerata) of the Compositae family, genus Mykania.

17. A composition for preventing gray hair which contains as an effective ingredient a plant extract wherein the effective ingredient is an extract from guaco misto (scientific name: Mykania Glomerata) of the Compositae family, genus Mykania.

18. A composition for use on the scalp which characteristically contains as an effective ingredient a Japanese pepper extract.

19. A tyrosinase activity promoting agent which contains as an effective ingredient a Japanese pepper extract.

20. The tyrosinase activity promoting agent of claim 19 wherein the tyrosinase activity promoting agent is a gray hair prevention agent.
